(19) Europäisches Patentamt
European Patent Office
Office européen des brevets

(11) Veröffentlichungsnummer: **0 126 028**
**A2**

(12) # EUROPÄISCHE PATENTANMELDUNG

(21) Anmeldenummer: **84810214.1**

(22) Anmeldetag: **07.05.84**

(51) Int. Cl.³: **C 07 D 211/46**

(30) Priorität: **11.05.83 CH 2591/83**
**27.06.83 CH 3495/83**

(43) Veröffentlichungstag der Anmeldung:
**21.11.84 Patentblatt 84/47**

(84) Benannte Vertragsstaaten:
**DE FR GB IT**

(71) Anmelder: **CIBA-GEIGY AG**
**Postfach**
**CH-4002 Basel(CH)**

(72) Erfinder: **Orban, Ivan, Dr.**
**Lehenmattstrasse 216**
**CH-4052 Basel(CH)**

(54) **Verfahren zur Herstellung von Polyestern aus aliphatischen Dicarbonsäuren und Polyalkylpiperidyldiolen.**

(57) Neues Verfahren zur Herstellung von Verbindungen der Formel I

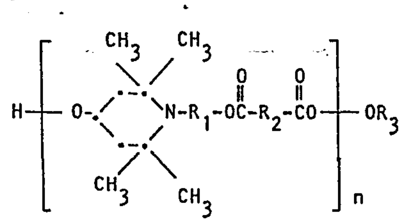

(I)

durch Polykondensation eines Diols der Formel II

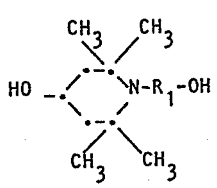

(II)

mit einem Dicarbonsäureester der Formel III

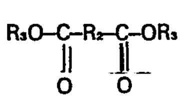

(III) .

Bezüglich der Bedeutung der Substituenten und Symbole $R_1$, $R_2$, $R_3$ und n wird auf Anspruch 1 verwiesen.

Das erfindungsgemässe Verfahren ist dadurch gekennzeichnet, dass das Reaktionsmedium in Gegenwart von mindestens 0,05 Mol%, bezogen auf das Diol der Formel II, einer metallorganischen Verbindung des Titans oder Zinns so lange bis das entstandene Methanol praktisch vollständig abdestilliert ist, bei einer maximalen Dampftemperatur von 75°C behandelt und danach noch mindestens 3 Stunden bei unter sukzessivem Zuführen des Lösungsmittels annähernd konstant gehaltenem Volumen der Reaktionslösung und bei einer Dampftemperatur zwischen 100 und 145°C weiterdestilliert, anschliessend eingedampft und die zurückgebliebene Schmelze nach dem Erstarren zerkleinert wird.

Man erhält so überraschenderweise in praktisch quantitativer Ausbeute Polyester der Formel I mit einem mittleren Molekulargewicht $\overline{M}n$ von mindestens 5000.

EP 0 126 028 A2

Croydon Printing Company Ltd.

CIBA-GEIGY AG
Basel (Schweiz)

3-14421/1+2/+

Verfahren zur Herstellung von Polyestern aus aliphatischen Dicarbonsäuren und Polyalkylpiperidyldiolen

Die vorliegende Erfindung betrifft ein neues Verfahren zur Herstellung von Polyestern aus aliphatischen Dicarbonsäuren und Polyalkylpiperidyldiolen durch Umsetzung eines entsprechenden Diols mit einer aliphatischen Dicarbonsäure unter distillativen Bedingungen aber bei konstantem Volumen der Reaktionslösung in Gegenwart von katalytischen Mengen eines geeigneten Katalysators.

Die Herstellung eines Polysuccinats von 1-(2-Hydroxyethyl)-2,2,6,6-tetramethyl-4-hydroxypiperidin ist bereits aus der DE-OS 27 19 131 (Beispiel 1) bekannt. Gemäss der dort beschriebenen Methode wird die Reaktion in Xylol in Gegenwart von mindestens 7 Mol% Katalysator bezogen auf die Piperidinverbindung bei 100 - 145°C, unter Abdestillieren des entstehenden Methanols durchgeführt. Dabei entsteht bei einer Endkonzentration des Produktes in der Reaktionslösung von ca. 65 Gew.-% ein Polysuccinat mit einem durchschnittlichen Molgewicht $\bar{M}n$ von maximal 4000. Das erhaltene gelbliche Produkt genügt hinsichtlich der Farbe nicht ganz den heutigen Erfordernissen der Technik.

Es ist nun gefunden worden, dass beim Durchführen der Reaktion ebenfalls unter Destillierbedingungen, aber bei durch sukzessive Zugabe von Lösungsmittel konstant gehaltenem Volumen der Reaktionslösung und bei Verwendung eines geeigneten Katalysators in bedeutend kleineren Mengen, überraschenderweise in praktisch quantitativer Ausbeute Polyester des obengenannten Typs mit einem höheren durchschnittlichen Molekulargewicht $\bar{M}n$ von mindestens 5000 erhalten werden,die bezüglich

der Farbe einwandfrei sind. Solche Polyester, die allgemein als wertvolle Stabilisatoren für organische Materialien bekannt sind, sind dank ihrem höheren $\bar{M}n$-Wert für bestimmte Anwendungen besonders geeignet.

Die vorliegende Erfindung betrifft demnach ein Verfahren zur Herstellung von Verbindungen der Formel I

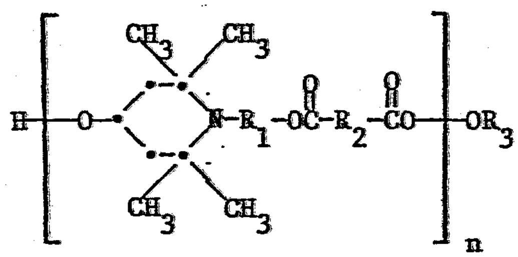

(I)

worin n eine Zahl zwischen 18 und 25, bevorzugt zwischen 19 und 22 bedeutet, $R_1$ und $R_2$ unabhängig voneinander $C_1$-$C_{18}$-Alkylen, durch -O-, -S- oder -N(R)- unterbrochenes $C_2$-$C_{18}$-Alkylen, wobei R Wasserstoff oder $C_1$-$C_4$-Alkyl bedeutet, $C_4$-$C_8$-Alkenylen sind und $R_3$ $C_1$-$C_4$-Alkyl bedeutet, durch Polykondensation eines Diols der Formel II

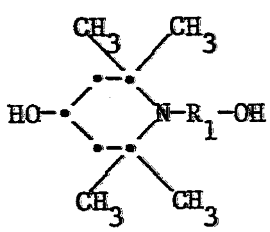

(II)

mit einem Dicarbonsäureester der Formel III

$$R_3O-\underset{\underset{O}{\|}}{C}-R_2-\underset{\underset{O}{\|}}{C}-OR_3 \qquad (III)$$

im Molverhältnis von ca. 1:1 in einem geeigneten Lösungsmittel, dadurch gekennzeichnet, dass man das Reaktionsmedium in Gegenwart von mindestens 0,05 Mol%, bezogen auf das Diol der Formel II, einer metallorganischen Verbindung des Titans oder Zinns so lange bis das entstandene Methanol praktisch vollständig abdestilliert ist, bei einer maximalen Dampftemperatur von 75°C, bevorzugt 69 bis 71°C, behandelt und danach noch mindestens 3 Stunden, bevorzugt 3 1/2 bis 5 Stunden bei unter sukzessivem Zuführen des Lösungsmittels annähernd konstant gehaltenem Volumen der Reaktionslösung und bei einer Dampftemperatur zwischen 100 und 145°C, bevorzugt zwischen 135 und 140°C, weiterdestilliert,

anschliessend eindampft und die zurückgebliebene Schmelze nach dem Erstarren zerkleinert.

Geeignete Katalysatoren sind z.B. solche der Formel IV und V

$(R_4O)_4$—Ti (IV), 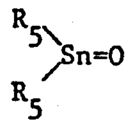 (V)

worin $R_4$ $C_1$-$C_{18}$-Alkyl, Phenyl oder Benzyl und $R_5$ $C_4$-$C_{18}$-Alkyl bedeuten. Bevorzugt werden Dibutylzinnoxid und insbesondere Tetrabutyl-o-titanat.

$R_1$ und $R_2$ sind als $C_1$-$C_{18}$-Alkylen, bevorzugt $C_2$-$C_6$-Alkylen, z.B. Methylen, Dimethylen, Trimethylen, Tetramethylen, Hexamethylen, 2,2-Dimethyl-trimethylen, Octamethylen, Nonamethylen, Decamethylen, Dodecamethylen oder Octadecamethylen. Bevorzugt ist Ethylen.

Als durch -O-, -S- oder -N(R)- unterbrochenes $C_2$-$C_{18}$-Alkylen bedeuten $R_1$ und $R_2$ beispielsweise 2-Thiapropylen-1,3, 3-Thiapentylen-1,5, 4-Oxaheptamethylen-1,7, 3,6-Dioxaoctamethylen-1,8 oder 3,6-Diazaoctamethylen-1,8.

Bedeuten etwaige Substituenten $C_1$-$C_4$-Alkyl so handelt es sich um Methyl, Ethyl, n-Propyl, Isopropyl, n-Butyl, sec.-Butyl oder tert.-Butyl. $R_4$ bedeutet als $C_1$-$C_{18}$-Alkyl zusätzlich z.B. n-Pentyl, 2,2-Dimethylpropyl, n-Hexyl, 2,3-Dimethylbutyl, n-Octyl, 1,1,3,3-Tetramethylbutyl, Nonyl, Decyl, Dodecyl, Hexadecyl oder Octadecyl. $R_5$ bedeutet als $C_4$-$C_{18}$-Alkyl z.B. n-Butyl, sec.-Butyl, tert.-Butyl, n-Pentyl, n-Hexyl, n-Octyl, 1,1,3,3-Tetramethylbutyl, n-Decyl, n-Dodecyl, n-Hexadecyl oder n-Octadecyl. $R_4$ und $R_5$ sind bevorzugt n-Butyl.

Sind $R_1$ und $R_2$ $C_4$-$C_8$-Alkenylen, so handelt es sich z.B. um 2-Butenylen-1,4.

Die Katalysatoren werden in Mengen von mindestens 0,05, bevorzugt aber zwischen 0,1 und 1,0 Mol% bezogen auf das Diol der Formel II eingesetzt.

Als Lösungsmittel sind inerte, insbesondere aromatische Lösungsmittel wie z.B. Toluol oder Xylol geeignet. Bevorzugt wird Xylol. Das Lösungsmittel wird in Mengen von 100 bis 200 Gew.-% bezogen auf das Diol der Formel II vorgelegt, wobei bei der sukzessiven Zuführung geachtet werden muss, dass das Volumen der Reaktionslösung annähernd konstant gehalten wird, d.h. mit einer Abweichung, welche 30, bevorzugt 15, insbesondere 10 Vol.-% nicht übersteigen soll.

Nach beendeter Reaktion ist es zweckmässig die Reaktionslösung zu filtrieren um eventuell vorhandene unlösliche Anteile und den Katalysator zu entfernen. Bei Verwendung eines im Lösungsmittel löslichen Katalysators, wie z.B. Tetrabutyl-o-titanat, geht man folgendermassen vor. Die Reaktionslösung wird bevorzugt auf 80 bis 100°C abgekühlt, mit 1 bis 3 Gew.% eines üblichen Filtrierhilfsmittels (z.B. ®Celite 545) und 10 bis 15 Gew.-% Wasser, bezogen auf das Diol der Formel II, versetzt und kurze Zeit am Rückfluss gehalten, dann azeotrop entwässert. Filtriert wird bevorzugt bei 120 - 130°C.

Durch das erfindungsgemässe Verfahren wird auch eine deutlich bessere Raum/Zeit-Ausbeute gewährleistet.

Von besonderem Interesse ist das Verfahren zur Herstellung von Verbindungen der Formel VI

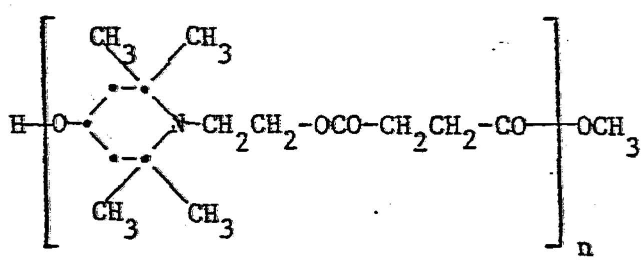

(VI),

worin n eine Zahl zwischen 18 und 25 bedeutet, durch Polykondensation von 1-(2-Hydroxyethyl)-2,2,6,6-tetramethyl-4-hydroxypiperidin mit Bernsteinsäuredimethylester im Molverhältnis von ca. 1:1 in einem geeigneten Lösungsmittel, dadurch gekennzeichnet, dass man das Reaktionsmedium in Gegenwart von Tetrabutyl-o-titanat so lange bis das entstandene

Methanol praktisch vollständig abdestilliert ist, bei einer maximalen Dampftemperatur von 75°C behandelt und danach noch mindestens 3 Stunden bei unter sukzessivem Zuführen des Lösungsmittels annähernd konstant gehaltenem Volumen der Reaktionslösung und bei einer Dampftemperatur zwischen 100 und 145°C weiterdestilliert, anschliessend eindampft und die zurückgebliebene Schmelze nach dem Erstarren zerkleinert.

Die Verbindungen der Formel I sind wertvolle Stabilisatoren für organische Materialien, die der Zersetzung unterworfen sind, vorzugsweise für synthetische Polymere.

Die folgenden Beispiele erläutern die Erfindung, ohne sie einzuschränken.

Beispiel 1: In einem 750 ml Kolben mit Destillationskolonne werden 171,1 g 1-(2-Hydroxyethyl)-2,2,6,6-tetramethyl-4-hydroxypiperidin und 225 g Xylol vorgelegt. Das Gemisch wird aufgeheizt und azeotrop entwässert, auf 130°C gekühlt und nacheinander mit 124,4 g Bernsteinsäuredimethylester und 0,8 g (0,28 Mol% bezogen auf das Hydroxypiperidinderivat) Tetrabutyl-orthotitanat versetzt.
Das entstehende Methanol wird bei einer auf max. 70°C geregelte Dampftemperatur abdestilliert. Nach 1,5 Stunden ist die Destillation praktisch beendet. Nun wird bei einer Dampftemperatur von ca. 137°C noch während 3,5 Stunden weiterdestilliert. Die Destillationsgeschwindigkeit wird dabei auf ca. 100 ml/h eingestellt. Das Volumen im Kolben wird durch sukzessive Zugabe von total ca. 350 ml Xylol konstant gehalten. Die Lösung wird auf 90°C gekühlt und mit 20 ml Wasser und 3 g Filterhilsmittel Celite 545 versetzt. Das Gemisch wird 15 Minuten am Rückfluss gehalten und anschliessend azeotrop entwässert. Die Suspension wird bei 120 - 130°C über eine Drucknutsche geklärt und das Klarfiltrat eingedampft, die zurückgebliebene Schmelze abgekühlt und nach dem Erstarren zerkleinert.

Man erhält so 240 g (99,8 % d.Th.) Polysuccinat als farbloses bis Spur gelbliches Pulver. $\overline{Mn}$-Wert 5500 - 6000. Erweichungspunkt ab 70°C.

Beispiel 2: Reaktion und Aufarbeitung werden analog Beispiel 1 durchgeführt, jedoch 5 Stunden bei Dampftemperatur von ca. 137°C bei konstant gehaltenem Volumen der Reaktionslösung destilliert. Das Ergebnis ist gleich wie im Beispiel 1, der $\overline{Mn}$-Wert beträgt jedoch 6500 - 7000.

Beispiel 3: Das Verfahren wird genau wie in Beispiel 1 wiederholt, mit der Ausnahme, dass anstelle von 0,8 g nur 0,2 g Tetrabutyl-orthotitanat (0,07 Mol% bezogen auf das Hydroxypiperidinderivat) eingesetzt werden und, dass statt 3,5 Stunden, 10 Stunden bei einer Dampftemperatur von 137°C weiterdestilliert wird. Man erhält mit praktisch quantitativer Ausbeute ein Produkt mit $\overline{Mn}$-Wert 10 500 - 11 000. Erweichungspunkt ab 106°C.

Beispiel 4: Das Verfahren wird genau wie in Beispiel 1 wiederholt, mit der Ausnahme, dass anstelle von 0,8 g Tetrabutyl-orthotitanat 1,0 g Dibutylzinnoxid (0,5 Mol% bezogen auf das Hydroxypiperidinderivat) eingesetzt und, dass statt 3,5 Stunden, 5 Stunden bei einer Dampftemperatur von 137°C weiterdestilliert wird. Man erhält mit praktisch quantitativer Ausbeute ein Produkt mit $\overline{Mn}$-Wert 13 000 - 13 500. Erweichungspunkt ab 120°C.

Beispiel 5: Man verfährt genau wie in Beispiel 1, mit der Ausnahme, dass anstelle von 0,8 g Tetrabutyl-orthotitanat 0,1 g Dibutylzinnoxid (0,05 Mol% bezogen auf das Hydroxypiperidinderivat) eingesetzt wird und, dass statt 3,5 Stunden bei 137°C, 10 Stunden bei 145°C Dampftemperatur weiterdestilliert wird. Man erhält mit praktisch quantitativer Ausbeute ein Produkt mit $\overline{Mn}$ 5500-6000. Erweichungspunkt ab 70°C.

Patentansprüche

1. Verfahren zur Herstellung von Verbindungen der Formel I

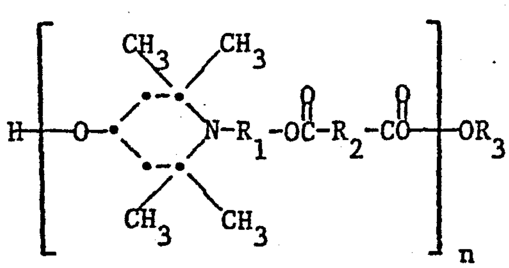

(I)

worin n eine Zahl zwischen 18 und 25, bevorzugt zwischen 19 und 22 bedeutet, $R_1$ und $R_2$ unabhängig voneinander $C_1$-$C_{18}$-Alkylen, durch -O-, -S- oder -N(R)- unterbrochenes $C_2$-$C_{18}$-Alkylen, wobei R Wasserstoff oder $C_1$-$C_4$-Alkyl bedeutet, $C_4$-$C_8$-Alkenylen sind und $R_3$ $C_1$-$C_4$-Alkyl bedeutet, durch Polykondensation eines Diols der Formel II

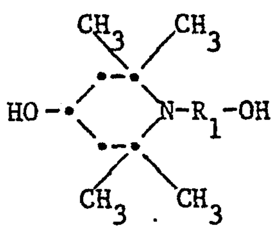

(II)

mit einem Dicarbonsäureester der Formel III

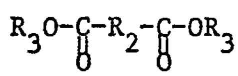

(III)

im Molverhältnis von ca. 1:1 in einem geeigneten Lösungsmittel, dadurch gekennzeichnet, dass man das Reaktionsmedium in Gegenwart von mindestens 0,05 Mol%, bezogen auf das Diol der Formel II, einer metallorganischen Verbindung des Titans oder Zinns so lange bis das entstandene Methanol praktisch vollständig abdestilliert ist, bei einer maximalen Dampftemperatur von 75°C behandelt und danach noch mindestens 3 Stunden bei unter sukzessivem Zuführen des Lösungsmittels annähernd konstant gehaltenem Volumen der Reaktionslösung und bei einer Dampftemperatur zwischen 100 und 145°C weiterdestilliert, anschliessend eindampft und die zurückgebliebene Schmelze nach dem Erstarren zerkleinert.

2. Verfahren gemäss Anspruch 1, zur Herstellung von Verbindungen der Formel VI

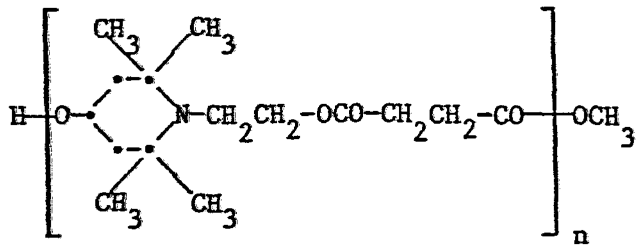 (VI),

worin n eine Zahl zwischen 18 und 25 bedeutet, durch Polykondensation von 1-(2-Hydroxyethyl)-2,2,6,6-tetramethyl-4-hydroxypiperidin mit Bernsteinsäuredimethylester im Molverhältnis von ca. 1:1 in einem geeigneten Lösungsmittel, dadurch gekennzeichnet, dass man das Reaktionsmedium in Gegenwart von Tetrabutyl-o-titanat so lange bis das entstandene Methanol praktisch vollständig abdestilliert ist, bei einer maximalen Dampftemperatur von 75°C behandelt und danach noch mindestens 3 Stunden bei unter sukzessivem Zuführen des Lösungsmittels annähernd konstant gehaltenem Volumen der Reaktionslösung und bei einer Dampftemperatur zwischen 100 und 145°C weiterdestilliert, anschliessend eindampft und die zurückgebliebene Schmelze nach dem Erstarren zerkleinert.

3. Verfahren gemäss Anspruch 1, dadurch gekennzeichnet, dass als Lösungsmittel Xylol verwendet wird.

4. Verfahren gemäss Anspruch 1, dadurch gekennzeichnet, dass als Katalysator Tetrabutyl-o-titanat verwendet wird.

5. Verfahren gemäss Anspruch 1, dadurch gekennzeichnet, dass man so lange bis das entstandene Methanol praktisch vollständig abdestilliert ist, bei einer Dampftemperatur von 69 bis 71°C behandelt.

6. Verfahren gemäss Anspruch 3, dadurch gekennzeichnet, dass nach dem Abdestillieren des entstandenen Methanols noch 3 1/2 bis 5 Stunden unter suksessivem Zuführen von Xylol bei einer Dampftemperatur zwischen 135 und 140°C weiterdestilliert wird.